**Europäisches Patentamt**

**European Patent Office**

**Office européen des brevets**

(11) Veröffentlichungsnummer: **0 103 810**
**B1**

(12) ## EUROPÄISCHE PATENTSCHRIFT

(45) Veröffentlichungstag der Patentschrift:
**28.01.87**

(51) Int. Cl.⁴: **C 07 C 45/50**, C 07 C 47/02

(21) Anmeldenummer: **83108791.1**

(22) Anmeldetag: **07.09.83**

(54) **Verfahren zur Herstellung von Aldehyden.**

(30) Priorität: **18.09.82 DE 3234701**

(43) Veröffentlichungstag der Anmeldung:
**28.03.84 Patentblatt 84/13**

(45) Bekanntmachung des Hinweises auf die Patenterteilung:
**28.01.87 Patentblatt 87/5**

(84) Benannte Vertragsstaaten:
**AT BE DE FR GB IT LU NL SE**

(56) Entgegenhaltungen:
**DE - B - 1 115 727**
**GB - A - 1 458 375**

(73) Patentinhaber: **Ruhrchemie Aktiengesellschaft,
Bruchstrasse 219, D-4200 Oberhausen 11 (DE)**

(72) Erfinder: **Cornlis, Boy, Dr. Dipl.-Chem.,
Friedrich-Ebert-Strasse 45, D-4220 Dinslaken (DE)**
Erfinder: **Hibbel, Josef, Dipl.-Ing., Bruchsteg 13,
D-4200 Oberhausen 11 (DE)**
Erfinder: **Konkol, Werner, Dr. Dipl.-Chem.,
Lützowstrasse 40a, D-4200 Oberhausen 11 (DE)**
Erfinder: **Lieder, Bernhard, Siegfriedstrasse 61,
D-4250 Bottrop (DE)**
Erfinder: **Much, Joachim, Dipl.-Ing., Im Torfveen 2,
D-4200 Oberhausen 14 (DE)**
Erfinder: **Schmidt, Volkmar, Dipl.-Ing., Lützowstrasse 51,
D-4200 Oberhausen 11 (DE)**
Erfinder: **Wiebus, Ernst, Ferdinandstrasse 77,
D-4200 Oberhausen 14 (DE)**

(74) Vertreter: **Reichelt, Karl-Heinz, Dr., m. Br. Ruhrchemie
Aktiengesellschaft Abt. PLD Postfach 13 01 60,
D-4200 Oberhausen 11 (DE)**

## Beschreibung

Die vorliegende Erfindung betrifft ein Verfahren zur Herstellung von Aldehyden durch Hydroformylierung von Olefinen in Gegenwart wasserlöslicher Rhodium-Komplexkatalysatoren.

Es ist bekannt, durch Umsetzung von Olefinen mit Synthesegas, d.h. einem Gemisch aus Kohlenmonoxid und Wasserstoff, Aldehyde und Alkohole herzustellen. Die Reaktion wird durch Hydridometallcarbonyle, vorzugsweise solcher der Metalle der 8. Gruppe des Periodensystems, katalysiert. Neben Kobalt, das als Katalysatormetall in grossem Umfang technische Anwendung findet, gewinnt Rhodium zunehmende Bedeutung. Im Gegensatz zu Kobalt gestattet Rhodium, die Reaktion bei niedrigem Druck durchzuführen; darüber hinaus werden vorzugsweise n-Aldehyde und nur in untergeordnetem Masse iso-Aldehyde gebildet. Schliesslich ist auch die Hydrierung der Olefine zu gesättigten Kohlenwasserstoffen bei Anwendung von Rhodium-Katalysatoren deutlich niedriger als bei Anwendung von Kobalt-Katalysatoren.

Bei den in der Technik eingeführten Verfahren wird der Rhodium-Katalysator in Form von Hydridorhodiumcarbonylen eingesetzt, die zusätzliche Liganden enthalten. Besonders bewährt haben sich als Liganden tertiäre Phosphine oder Phosphite. Ihre Anwendung ermöglicht es, den Reaktionsdruck auf Werte unter 30 000 kPa zu senken.

Als unbefriedigend erweist sich bei diesem Verfahren die Abtrennung der Reaktionsprodukte von den homogen im Reaktionsprodukt gelösten Katalysatoren. Im allgemeinen wird hierzu das Umsetzungsprodukt aus dem Reaktionsgemisch abdestilliert. In der Praxis kann dieser Weg aber nur bei der Hydroformylierung niedriger Olefine bis zu etwa Penten beschritten werden. Ausserdem hat sich gezeigt, dass die hiermit verbundene thermische Belastung zu erheblichen Katalysatorverlusten durch Zersetzung der Rhodiumkomplexverbindungen führt.

Die geschilderten Mängel werden durch Anwendung von Katalysatorsystemen vermieden, die in Wasser löslich sind. Derartige Katalysatoren sind z.B. in der DE-PS 26 27 354 beschrieben. Die Löslichkeit der Rhodiumkomplexverbindungen wird hierbei durch Verwendung von trisulfonierten Triarylphosphinen als Komplexbestandteil erreicht. Die Abtrennung des Katalysators vom Reaktionsprodukt nach der Hydroformylierung erfolgt in diesem Fall einfach durch Trennung von wässriger und organischer Phase, d.h. ohne zusätzliche, für die Destillation der Reaktionsprodukte notwendige thermische Belastung des Reaktionsgemisches. Allerdings beschränken sich die Angaben in der genannten DE-PS auf einen diskontinuierlichen Prozess in Autoklaven, der für eine wirtschaftliche Nutzung nicht geeignet ist. Nach einem weiteren Prozess erreicht man die Löslichkeit des Katalysatorsystems in Wasser durch Verwendung ein- oder mehrfach carboxylierter Arylphosphinliganden.

Es bestand daher die Aufgabe, ein Verfahren zur Herstellung von Aldehyden der vorgenannten Art zu entwickeln, das technisch mit Erfolg durchzuführen ist.

Die Erfindung besteht in einem kontinuierlichen Verfahren zur Herstellung von Aldehyden durch Umsetzung von Olefinen mit Kohlenmonoxid und Wasserstoff in Gegenwart von Wasser und wasserlöslichen Rhodium-Phosphin-Komplexverbindungen bei erhöhter Temperatur und Drücken von 100 bis 30 000 kPa. Es ist dadurch gekennzeichnet, dass die Reaktanten innig vermischt und bei Temperaturen von 90 bis 150°C zur Reaktion gebracht werden, wobei der Anteil der gasförmigen Bestandteile in der flüssigen Phase auf 5 bis 30 Vol.%, bezogen auf die Mischphase, eingestellt wird, das Volum-Verhältnis von wässriger zu organischer Phase 1:1 bis 100:1 beträgt und zunächst flüssige Phase und gasförmige Phase, sodann die flüssige Phase in wässrige und organische Anteile jeweils ohne vorherige Kühlung getrennt werden und die Reaktionswärme abgeführt wird.

Die neue Arbeitsweise stellt nicht nur sicher, dass das Katalysatorsystem unter schonenden Bedingungen abgetrennt und in den Prozess wieder eingesetzt wird, sondern erlaubt auch die Durchführung der Umsetzung unter technisch und wirtschaftlich optimalen Bedingungen. Besondere Bedeutung kommt der Tatsache zu, dass der erfindungsgemässe Prozess in einfacher Weise die Gewinnung der Reaktionswärme ermöglicht, ohne dass die Lebensdauer der Katalysatoren beeinträchtigt wird.

Die Abführung der Reaktionswärme kann in bekannter Weise mittels Wasser oder Wasserdampf erfolgen. Besonders bewährt hat es sich, die Reaktionswärme ohne Vermittlung eines Hilfsmediums unmittelbar auf die Produkte der Hydroformylierung zu übertragen, um sie zu verdampfen und unter Verwendung der Reaktionswärme destillativ zu reinigen. Die erfindungsgemässe Arbeitsweise eignet sich insbesondere zur Überführung von Olefinen mit 2 bis 15 Kohlenstoffatomen in die um ein Kohlenstoffatom reicheren Aldehyde bzw. Alkohole. Die Reaktanten, d.h. Olefin und Synthesegas, werden dem Reaktor gemeinsam oder getrennt zugeführt. Zweckmässig ist es, die Reaktanten vorzuwärmen, vorzugsweise auf die Temperatur, bei der die Umsetzung abläuft. Es hat sich bewährt, die Vorwärmung mit Prozessabwärme vorzunehmen, z.B. der Wärme, die bei der Kondensation der Reaktionsprodukte im Verlauf der destillativen Reinigung zurückgewonnen werden kann.

Die Umsetzung der Ausgangsstoffe im Reaktor erfolgt bei Temperaturen von 90 bis 150°C. Durch den Entfall der zusätzlichen thermischen Belastung zur destillativen Entfernung der Reaktanten wird der Katalysator auch im Dauerbetrieb kaum desaktiviert. Geringfügige Schädigungen der Katalysatoren halten sich in wirtschaftlich vertretbaren Grenzen.

Die Umsetzung der Reaktionspartner im Reaktor erfolgt in einem aus flüssiger und gasförmiger Phase bestehenden System. Hierbei besteht die flüssige Phase aus zwei ineinander nicht oder nur sehr wenig löslichen Komponenten, der wässrigen Katalysatorlösung, gegebenenfalls dem flüssigen Olefin und dem flüssigen organischen Reaktionsprodukt, das gegebenenfalls noch ein Lösungsmittel enthalten kann. Massgebend für den Ablauf der Reaktion ist, dass die wässrige Phase mit den gasförmigen Reaktanten gesättigt ist. Um dies zu erreichen, ist es erforderlich, für eine möglichst grosse Berührungsfläche zwischen der flüssigen – also aus wässriger und organischer Komponente bestehenden – und der gasförmigen Phase zu sorgen, so dass der Anteil der gasförmigen Bestandteile in der flüssigen Phase auf 5 bis 30 Vol.%, bezogen auf die Mischphase, eingestellt wird. Dieses Ziel kann auf verschiedenen Wegen erreicht werden. Nach einer bewährten Ausführungsform der Erfindung leitet man die gasförmigen Ausgangsstoffe, d.h. Olefin und Synthesegas, dem intensiv gerührten Reaktorinhalt zu. Nach einer anderen Verfahrensvariante führt man die gasförmigen Reaktionspartner dem flüssigen Reaktorinhalt über entsprechende Verteilungsvorrichtungen zu. Geeignet sind z.B. Siebböden oder Fritten. Es ist auch möglich, Rührung und Verteilung der gasförmigen Reaktionspartner miteinander zu kombinieren, z.B. durch Verwendung eines Begasungsrührers.

Ein sehr wesentlicher Aspekt der vorliegenden Erfindung ist es, den Anteil der organischen Phase im Reaktionsgemisch gering zu halten. Überraschenderweise trägt nämlich die organische Phase zur Löslichkeit der Reaktanten in der wässrigen Phase nicht bei. Dadurch gelingt es auch, zu vermeiden, dass das Reaktionsprodukt unerwünschte Nebenreaktionen eingeht, die bei zunehmender Verweilzeit des Produktes im Reaktor nicht auszuschliessen sind.

Dementsprechend beträgt erfindungsgemäss das Volumverhältnis von wässriger zu organischer Phase 1:1 bis 100:1, vorzugsweise 10:1 bis 100:1. Zur Einstellung des genannten Volumverhältnisses bieten sich verschiedene Wege an. Nach einer bewährten Ausführungsform der neuen Arbeitsweise wird ein solcher Teil des Reaktionsgemisches aus dem Reaktor ausgeschleust und einer Phasentrennung unterworfen, dass sich nach Rückführung der wässrigen Phase das gewünschte Volumverhältnis im Reaktor ausbildet.

Die Phasentrennung kann nach einer anderen Ausführungsform der Erfindung auch innerhalb des Reaktors in einer Beruhigungszone vorgenommen werden. Die vorstehend beschriebene Phasentrennung erfolgt nach dem neuen Verfahren in jedem Fall ohne vorherige Kühlung des Reaktionsgemisches. Durch diese Massnahme wird erreicht, dass die gasförmigen Olefine in den unter den gegebenen Bedingungen flüssigen Bestandteilen des Reaktionsgemisches nur in kleinen Mengen gelöst und mit dem Reaktionsprodukt ausgetragen werden.

Das für die Hydroformylierung verwendete Synthesegas enthält Kohlenmonoxid und Wasserstoff zweckmässigerweise im Vol.-Verhältnis 1:1. Es ist aber möglich, dieses Verhältnis zur Erreichung bestimmter Effekte, z.B. zur Steigerung der Reaktionsgeschwindigkeit, zu variieren.

Als Katalysatoren werden komplexe Verbindungen des Rhodiums eingesetzt, die neben Kohlenmonoxid und Wasserstoff sulfonierte oder carboxylierte Phosphine enthalten. Derartige Phosphine leiten sich insbesondere von Triarylphosphinen ab, wobei unter Arylrest vorzugsweise der Phenylrest und der Naphthylrest verstanden wird. Es ist nicht erforderlich, dass alle drei Arylreste Sulfonsäuregruppen oder Carboxylgruppen tragen. Es hat sich gezeigt, dass bereits eine Sulfonsäuregruppe oder eine Carboxylgruppe im Phosphinmolekül der Komplexverbindung eine ausreichende Wasserlöslichkeit verleiht. Der Katalysator kann dem Reaktionsgemisch präformiert zugesetzt werden. Es ist aber auch möglich, ihn in situ zu bilden. Üblicherweise setzt man Rhodium in einer Menge von 50 bis 800 ppm, bezogen auf die wässrige Katalysatorlösung, zu. Das sulfonierte oder carboxylierte Triarylphosphin muss, bezogen auf den Rhodiumkomplex, im Überschuss vorhanden sein. Besonders bewährt hat es sich, je g-Atom Rhodium 10 bis 100 g Moleküle sulfoniertes oder carboxyliertes Phosphin zuzusetzen. Die Reaktionsdrücke bewegen sich im Bereich von 100 bis 30 000 kPa Kohlenmonoxid und Wasserstoff.

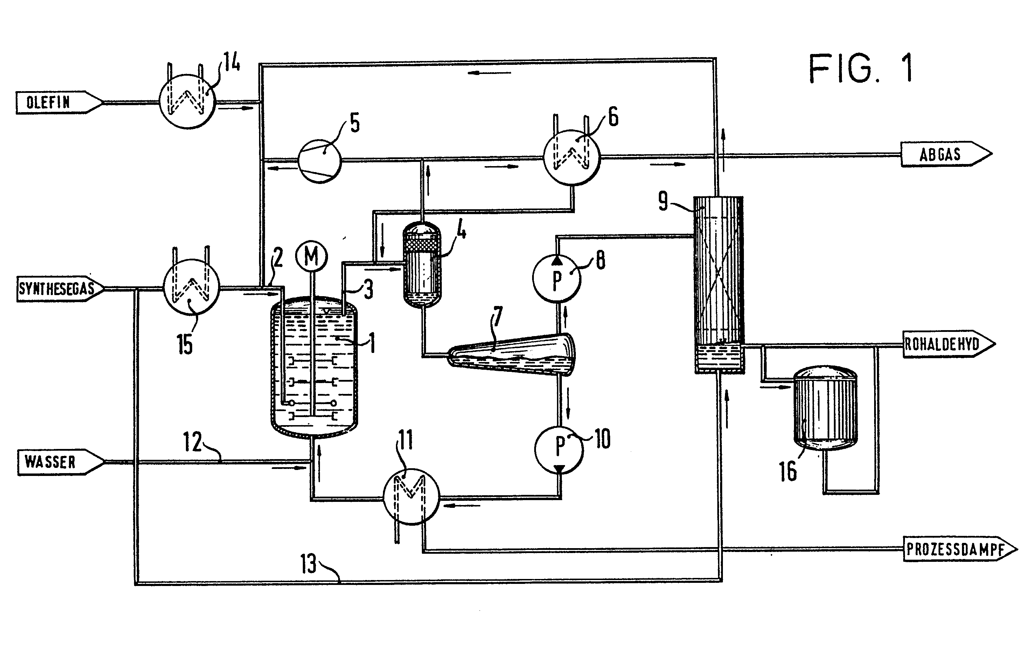

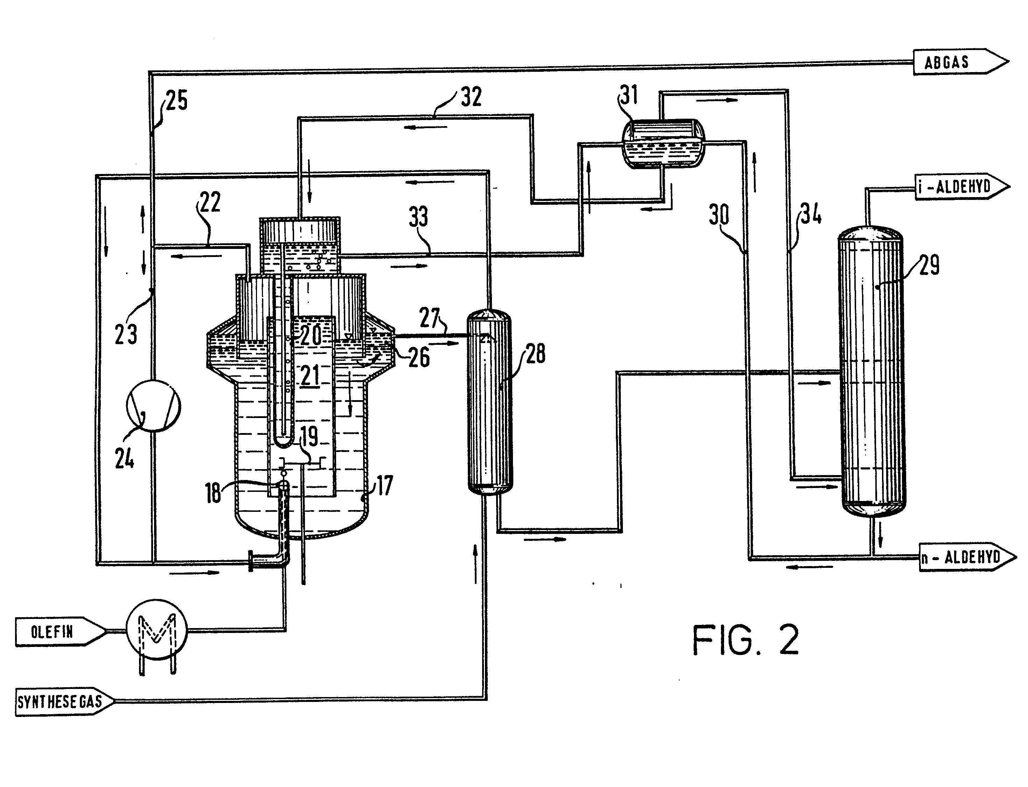

Die Durchführung der Umsetzung nach dem erfindungsgemässen Verfahren ist in der Zeichnung 1 schematisch dargestellt.

In einem Reaktor 1 wird das in Wasser gelöste Katalysatorsystem vorgelegt. Olefin und Synthesegas werden dem Reaktor 1 über Leitungen 2 zugeführt und unter inniger Vermischung zur Reaktion gebracht. Über einen Tauchstutzen 3 verlässt das Reaktionsprodukt, in Mischung mit wässriger Katalysatorlösung, nicht umgesetztem Synthesegas und Olefin, den Reaktor. In einem Trenngefäss 4 wird die Gasphase – im wesentlichen Synthesegas, Olefin und aus dem Olefin gebildeter gesättigter Kohlenwasserstoff – von den Flüssigprodukten getrennt und über einen Kreislaufkompressor 5 wieder dem Reaktor zugeführt. Eine Teilmenge des Kreislaufgases wird in einem Kühler 6 von den kondensierbaren Reaktionsprodukten befreit und in das Abgassystem abgegeben. Die im Trenngefäss 4 abgeschiedene heisse Flüssigkeit wird ohne vorherige Kühlung einem Phasentrenner 7 zugeführt. In diesem trennt sich das rohe organische Reaktionsprodukt sehr leicht von der wässrigen Katalysatorphase. Durch den Verzicht, die organische Phase vor Abtrennung der unumgesetzten Reaktanten nicht zu kühlen, wird die erforderliche Rezirkulierung der gasförmigen Olefine über eine Strippkolonne 9 erheblich erleichtert. Denn im heissen organischen Reaktionsprodukt (dem Rohaldehyd), ist ihre Löslichkeit wesentlich geringer als im abgekühlten Reaktionsprodukt. Während das organische Reaktionsprodukt mittels einer Pumpe 8 der Strippkolonne 9 zugeführt wird, fördert eine Pumpe 10 die wässrige Katalysatorphase wieder zurück in den Reaktor 1, wobei in einem Wärmetauscher 11 die Wärme der exothermen Reaktion unter Erzeugung von Prozessdampf abgegeben wird. Mit der gekühlten Katalysatorphase wird Wasser über eine Leitung 12 dem Reaktor zugeführt, um den entsprechenden Wasseraustrag über das Abgas und über das Oxorohprodukt zu kompensieren. Das der Strippkolonne 9 zuströmende heisse Oxorohprodukt wird im Gegenstrom zu einer Synthesegasteilmenge geführt, die der Kolonne über eine Leitung 13 zuströmt. Das Synthesegas wird dabei mit dem im Oxorohprodukt gelösten Olefin beladen. Mit erhöhter Temperatur wird das Synthesegas dem Reaktor 1 zugeführt; ein weiterer Synthesegasteilstrom wird in einem Wärmetauscher 15 mit Prozesswärme vorerhitzt. Auch das Frischolefin wird vor Eintritt in den Reaktor mit Abwärme der Aldehyddestillation in einem Wärmetauscher 14 vorgewärmt und verdampft, während das Oxorohprodukt aus der Strippkolonne 9 ungekühlt direkt der Destillation zugeführt wird. Ein Pufferbehälter dient nur im Störungsfalle der Produktzwischenlagerung.

Eine weitere, technisch besonders zweckmässige Ausführungsformd des erfindungsgemässen Verfahrens ist in Zeichnung 2 dargestellt.

In einem Reaktor 17 wird die wässrige Katalysatorlösung vorgelegt. Durch als Vorverteiler dienende Doppelbrausen 18 erfolgt der Einsatz von Synthesegas und Olefin. Ein Begasungsrührer 19 sorgt für die Feinverteilung der Reaktanten. Die Wärme der exothermen Reaktion wird über ein Kühlregister 20 abgeleitet. In einem Führungsrohr 21 des Reaktors steigen die flüssigen und gasförmigen Komponenten auf und trennen sich am oberen Ende des Führungsrohres. Die gasförmigen Komponenten werden über eine Leitung 23 und ein Kreisgasverdichter 24 wieder in den Reaktor zurück- oder über eine Leitung 25 als Abgas abgeführt. Die wässrige Katalysatorlösung trennt sich in einem Ringraumabschneider 26 von dem gebildeten organischen Rohprodukt. Über eine Leitung 27 wird das Rohprodukt einer Strippkolonne 28 zugeführt und dort durch das im Gegenstrom geführte Synthesegas vom gelösten Olefin befreit. Das olefinfreie Rohprodukt wird in einer Kolonne 29 in die n- und iso-Komponenten zerlegt. Die für die Destillation erforderliche Wärme wird unmittelbar über das Kühlregister 20 gewonnen, in dem flüssiger Aldehyd aus dem Sumpf der Kolonne 29 über eine Leitung 30, einen Phasentrenner 31 und eine Leitung 32 in das Kühlregister geleitet wird und dort verdampft und über eine Leitung 33, den Phasentrenner 31 und eine Leitung 34 als dampfförmiger Aldehyd wieder der Kolonne 29 zugeführt wird.

Beispiel 1 (Vergleich)

In einem Reaktor wird unter Reaktionsbedingungen 50 l einer durch intensives Rühren homogenisierten Mischphase aus wässriger Katalysatorlösung, Oxorohprodukt, Synthesegas und Propylen aufrecht erhalten. Der kontinuierlich abgezogene, Katalysatorlösung, Oxorohprodukt, überschüssiges Propylen und Synthesegas enthaltende Produktstrom wird vor Produkttrennung gekühlt. Im gekühlten Oxorohprodukt sind unter den gewählten Bedingungen etwa 4,5 kg Propylen/kg Oxorohprodukt gelöst. Die Dichte dieser organischen Phase beträgt 0,6 g/cm$^3$. Der in der wässrigen Phase gelöste Propylenanteil ist vernachlässigbar gering. Der Dichteunterschied zwischen wässriger Phase (Dichte = 1,15 g/cm$^3$) und organischer Phase ist 0,55 g/cm$^3$. Dementsprechend trennen sich die beiden Phasen schnell und vollständig voneinander.

Beispiel 2

Die Umsetzung erfolgt unter den Bedingungen des Beispiel 1. Abweichend von Beispiels 1 wird jedoch der abgezogene Produktstrom aus Katalysatorlösung, Oxorohprodukt, überschüssigem Propylen und Synthesegas vor der Produkttrennung nicht gekühlt, sondern auf Reaktionstemperatur gehalten. Im Oxorohprodukt sind dann nur noch 0,4 kg Propylen/kg Oxorohprodukt gelöst. Dadurch stellt sich eine höhere Durchschnittsdichte der organischen Phase von 0,75 g/cm$^3$ ein, so dass sich der Dichteunterschied zur wässrigen Phase auf 0,4 g/cm$^3$ reduziert.

Überraschenderweise trennen sich trotz des geringen Dichteunterschiedes die Phasen ebenso schnell und vollständig voneinander wie nach der Arbeitsweise des Beispiels 1. Die Möglichkeit, organische und wässrige Phase auch ohne vorherige Abkühlung wirtschaftlich befriedigend und technisch einfach zu trennen, erleichtert die Kreislaufführung des Propylens erheblich. Denn die über die Strippkolonne 9 abzutrennende und in den Reaktor 1 zurückzuführende Propylenmenge wird von 4,5 auf 0,4 kg/kg Oxorohprodukt vermindert mit der Folge einer deutlichen Energieersparnis.

Beispiel 3

Im Reaktor 1 wird unter Reaktionsbedingungen 50 l einer durch intensives Rühren homogenisierten Mischphase aus wässriger Katalysatorlösung, Oxorohprodukt, Syntesegas und Propylen aufrecht erhalten. Zur Einstellung der Phasenverteilung zwischen wässriger und organischer Phase sowie zur Abführung der Reaktionswärme mittels der Kreislaufpumpe 10 werden je Stunde 100 l wässrige Katalysatorlösung im Kreis geführt. Unter den gewählten Reaktionsbedingungen bilden sich aus Propylen je Stunde 10 l Rohaldehyd, so dass das Verhältnis von wässriger zu organischer Phase 10:1 beträgt.

Die mittlere Verweilzeit des Rohaldehyds im Reaktor ist 27 Minuten. Im Oxorohprodukt sind 0,5 Gew.% unerwünschte, hochsiedende Nebenprodukte enthalten.

Beispiel 4

Es wird unter den Reaktionsbedingungen des Beispiels 1 gearbeitet, jedoch werden je Stunde nur 35 l Katalysatorlösung im Kreis geführt. Dadurch erhöht sich die mittlere Verweilzeit des Rohaldehyds im Reaktor auf 69 Min. Unter diesen Bedingungen erhält das Oxorohprodukt etwas 1,5 Gew.% unerwünschte, hochsiedende Nebenprodukte.

Bei einem Reaktor mit innenliegendem Kühlregister ohne Katalysatorkreislauf wird die Drehzahl des Rührers in Abhängigkeit vom Rührorgan so gewählt, dass die Mischphase in der gerührten Zone des Reaktors $\leq$ 10% Rohaldehyd homogen verteilt enthält und die in der Beruhigungszone auf der wässrigen Katalysatorlösung aufschwimmende Aldehydphase durch entsprechende Niveauhaltung gering ist, so dass auf diese Weise ebenfalls Verweilzeiten des Rohaldehyds im Syntheseteil von $\leq$ 30 Minuten eingehalten werden können.

Überraschend ist, dass die Verringerung der Drehzahl, die eine Entmischung der organischen und wässrigen Phase im oberen Teil des Reaktors ermöglicht, keinen negativen Einfluss auf die Durchsatzleistung des Reaktors hat. Offensichtlich wird in der intensiv gerührten Zone des Reaktors eine ausreichende Sättigung der Reaktanten in der wässrigen Katalysatorphase erreicht.

## Patentansprüche

1. Verfahren zur Herstellung von Aldehyden durch Umsetzung von Olefinen mit Kohlenmonoxid und Wasserstoff in Gegenwart von Wasser und wasserlöslichen Rhodium-Phosphin-Komplexverbindungen bei erhöhter Temperatur und Drücken von 100 bis 30 000 kPa, dadurch gekennzeichnet, dass die Reaktanten innig vermischt und bei Temperaturen von 90 bis 150°C zur Reaktion gebracht werden, wobei der Anteil der gasförmigen Bestandteile in der flüssigen Phase auf 5 bis 30 Vol.%, bezogen auf die Mischphase, eingestellt wird, das Volumverhältnis von wässriger zu organischer Phase 1:1 bis 100:1 beträgt und zunächst flüssige Phase und gasförmige Phase, sodann die flüssige Phase in wässrige und organische Anteile jeweils ohne vorherige Kühlung getrennt werden und die Reaktionswärme abgeführt wird.

2. Verfahren nach Anspruch 1, dadurch gekennzeichnet, dass die Reaktanten vor der Reaktion auf die Umsetzungstemperatur vorgewärmt werden.

3. Verfahren nach Anspruch 2, dadurch gekennzeichnet, dass die Vorwärmung mit Prozessabwärme erfolgt.

4. Verfahren nach Anspruch 1 bis 3, dadurch gekennzeichnet, dass die gasförmigen Ausgangsstoffe dem intensiv gerührten Reaktorinhalt zugeführt werden.

5. Verfahren nach Anspruch 1 bis 3, dadurch gekennzeichnet, dass die gasförmigen Ausgangsstoffe dem Reaktor über Verteilungsvorrichtungen zugeführt werden.

6. Verfahren nach Anspruch 1 bis 5, dadurch gekennzeichnet, dass das Volumverhältnis von Wasser zu organischer Phase 10:1 bis 100:1 beträgt.

7. Verfahren nach Anspruch 1 bis 6, dadurch gekennzeichnet, dass die Abführung der Reaktionswärme durch Vermittlung eines Hilfsmediums, wie Wasser oder Wasserdampf, erfolgt.

8. Verfahren nach Anspruch 1 bis 6, dadurch gekennzeichnet, dass die Abführung der Reaktionswärme ohne Vermittlung eines Hilfsmediums durch Verdampfung zumindest eines Teils der Reaktionsprodukte erfolgt.

9. Verfahren nach Anspruch 1 bis 8, dadurch gekennzeichnet, dass die Trennung der flüssigen Phase in wässrige und organische Anteile in einer Beruhigungszone im Reaktor erfolgt.

## Claims

1. A process for the preparation of aldehydes by the reaction of olefins with carbon monoxide and hydrogen in the presence of water and water-soluble rhodium phosphine complex compounds at elevated temperature and pressures of 100 to 30,000 kPa, characterised in that the reactants are homogeneously mixed and brought to reaction at temperatures of 90 to 150°C, whereby the proportion of gaseous components in the liquid phase is adjusted to 5 to 30 vol.%, based on the mixed phase, the volume ratio of aqueous to organic phase being 1:1 to 100:1 and the liquid phase and the gaseous phase are first separated and then the liquid phase is divided into aqueous and organic parts in each case without any previous cooling and the heat of reaction is removed.

2. A process according to claim 1, characterised in that before the reaction the reactants are preheated to the reaction temperature.

3. A process according to claim 2, characterised in that preheating takes place with process waste heat.

4. A process according to claims 1 to 3, characterised in that the gaseous starting materials are led into the intensively stirred reactor contents.

5. A process according to claims 1 to 3, characterised in that the gaseous starting materials are led into the reactor by means of distribution devices.

6. A process according to claims 1 to 5, characterised in that the volume ratio of water to organic phase is 10:1 to 100:1.

7. A process according to claims 1 to 6, characterised in that the heat of reaction is removed with the aid of an auxiliary medium such as water or steam.

8. A process according to claims 1 to 6, characterised in that the heat of reaction is removed without the aid of an auxiliary medium by the vaporisation of at least part of the reaction products.

9. A process according to claims 1 to 8, characterised in that the liquid phase is separated into aqueous and organic parts in a contact zone in the reactor.

## Revendications

1. Procédé de préparation d'aldéhydes par réaction d'oléfines avec de l'oxyde de carbone et de l'hydrogène en présence d'eau et de complexes rhodium-phosphines solubles dans l'eau à température élevée et sous des pressions de 100 à 30 000 kPa, caractérisé en ce que l'on mélange intimement les réactifs et on les fait réagir à des températures de 90 à 150°C en réglant la proportion des constituants gazeux dans la phase liquide à un niveau de 5 à 30% en volume, par rapport à la phase mélangée, avec un rapport en volume de 1:1 à 100:1 entre la phase aqueuse et la phase organique, et on sépare d'abord la phase liquide et la phase gazeuse puis la phase liquide en fractions aqueuse et organique, dans les deux cas sans refroidissement préalable, et on évacue la chaleur de réaction.

2. Procédé selon la revendication 1, caractérisé en ce que les réactifs sont chauffés à la température de réaction avant la réaction.

3. Procédé selon la revendication 2, caractérisé en ce que le réchauffage préalable est réalisé à l'aide de la chaleur évacuée dans l'opération.

4. Procédé selon les revendications 1 à 3, caractérisé en ce que les composants de départ gazeux sont envoyés dans le contenu du réacteur soumis à agitation intensive.

5. Procédé selon les revendications 1 à 3, caractérisé en ce que les produits de départ gazeux sont envoyés dans le réacteur par l'intermédiaire de dispositifs de répartition.

6. Procédé selon les revendications 1 à 5, caractérisé en ce que le rapport en volume entre la phase aqueuse et la phase organique va de 10:1 à 100:1.

7. Procédé selon les revendications 1 à 6, caractérisé en ce que la chaleur de réaction est évacuée par utilisation d'un fluide auxiliaire tel que l'eau ou la vapeur d'eau.

8. Procédé selon les revendications 1 à 6, caractérisé en ce que la chaleur de réaction est évacuée sans utilisation d'un fluide auxiliaire par vaporisation d'une partie au moins des produits de réaction.

9. Procédé selon les revendications 1 à 8, caractérisé en ce que la séparation de la phase liquide en une fraction aqueuse et une fraction organique est réalisée dans une zone tranquille du réacteur.

FIG. 1

OLEFIN
SYNTHESEGAS
WASSER
ABGAS
ROHALDEHYD
PROZESSDAMPF

0 103 810

FIG. 2

ABGAS

i -ALDEHYD

n -ALDEHYD

OLEFIN

SYNTHESE GAS

0103810